# EUROPÄISCHE PATENTSCHRIFT

(11) **EP 3 099 269 B1**
(45) Veröffentlichungstag und Bekanntmachung des Hinweises auf die Patenterteilung: **16.06.2021**
(21) Anmeldenummer: 15701535.5
(22) Anmeldetag: 28.01.2015
(51) Int. Cl.: A61C 13/01, A61C 19/10, G01J 3/50, A61C 8/00, A61C 13/10, A61C 13/225, A61C 13/271

(54) **GINGIVA-INDEXIERUNGSVORRICHTUNG UND VERFAHREN ZUR INDEXIERUNG DER GINGIVA**
GINGIVAL INDEXING DEVICE AND METHOD FOR INDEXING THE GINGIVA
DISPOSITIF D'INDEXATION GINGIVALE ET PROCÉDÉ D'INDEXATION GINGIVALE

(30) Priorität: 29.01.2014 DE 102014101059
(43) Veröffentlichungstag der Anmeldung: 07.12.2016
(73) Patentinhaber: Kulzer GmbH, 63450 Hanau (DE)
(72) Erfinder: REAY, David J., Nocton Lincoln LN4 2BE (GB); KUBIAK-ESSMANN, Harald, 61130 Nidderau (DE)
(74) Vertreter: Bendele, Tanja
(86) Internationale Anmeldenummer: PCT/EP2015/051693
(87) Internationale Veröffentlichungsnummer: WO 2015/113999

(56) Entgegenhaltungen:
- EP-A1- 2 674 129
- EP-A1- 2 676 633
- DE-A1-102009 023 952
- DE-U1-202005 021 152
- JP-A- H0 678 937
- US-A- 1 842 923
- US-A- 5 904 481
- US-A1- 2003 049 585
- US-A1- 2008 026 340
- US-A1- 2013 034 823

## Beschreibung

Die Erfindung betrifft eine Gingiva-Indexierungsvorrichtung mit verschiedenen, definierten roten Farbregionen (Rottönen) sowie ein Verfahren zur Erfassung der Gingiva-Situation, insbesondere zur Herstellung einer Gingiva-Restauration, umfassend eine Analyse mindestens eines Bereiches der Gingiva-Situation in Bezug auf ihre roten Farbtöne, die Position der roten Farbtöne, den Farbverlauf der roten Farbtöne, deren Geometrie und/oder deren Morphologie, wobei die Analyse manuell und/oder digital erfolgt, und Gingiva-Informationen erhalten werden, insbesondere in Form einer Gingiva-Kartierung. Optional wird ein virtuelles oder reales Modell einer Gingiva-Restauration als Nachbildung der fehlenden Zahnfleischpartie bzw. Gingiva-Partie hergestellt. Eine Gingiva-Restauration wird auch als Zahnfleischmaske bezeichnet.

Bei den Gingiva-Restaurationen bzw. Zahnfleischmasken unterscheidet man verschiedene Typen. Es gibt Zahnfleischmasken zum Einsatz im Patientenmund als Ersatz für alters- oder krankheitsbedingten Zahnfleischrückgang sowie Zahnfleischmasken als Hilfsmittel für den Zahntechniker, um Präparationsgrenzen auf dem Gipsmodell zu simulieren. Ebenso gibt es Gingiva-Restaurationen bzw. Zahnfleischmasken als Teil einer Zahnprothese. Die Zahnprothese kann festsitzenden oder herausnehmbaren Zahnersatz umfassen. Dabei kann der herausnehmbare Zahnersatz beispielsweise auf in den Kiefer eingebrachten Implantaten und darauf angebrachten Halteelementen, wie Druckknöpfen oder Stegen, fixiert werden. Ein Nachteil dieser Zahnprothesen mit Zahnersatz ist die Einfarbigkeit der Gingiva-Restauration an der Zahnprothese, die den Rückgang der Gingiva und optional des Kieferschwundes (Atropie) kaschieren sollen.

Typ 1: Zahnfleischmasken können bei krankheits- oder altersbedingtem Zahnfleischrückgang zum Einsatz kommen. Hier zeigen sich häufig durch die zunehmende Sichtbarkeit der Zahnhälse unnatürlich lange Zähne, dies kann zu schweren kosmetischen Beeinträchtigungen führen. Handelt es sich um einen krankheitsbedingten Zahnfleischrückgang, so steht die Behandlung der Grunderkrankung im Vordergrund. Ist ein stabiles und entzündungsfreies Zahnfleisch wiederhergestellt, so kann der Rückgang des Zahnfleisches durch eine sogenannte Zahnfleischmaske kaschiert werden. Bei einer Zahnfleischmaske handelt es sich um ein künstliches Zahnfleisch, das üblicherweise aus einem flexiblen Polymer besteht. Die Zahnfleischmaske ist eine herausnehmbare Zahnfleischimitation, die in der Regel vom Patienten selbst eingesetzt und entfernt werden kann. Auch ein Zahnfleischrückgang bzw. Gingiva-Defizit im Gebiss mit Zahnimplantaten kann durch eine Zahnfleischmaske bzw. Gingiva-Restauration kaschiert werden. In diesem Fall kann die Gingiva-Restauration Teil einer prothetischen Versorgung mit Zahnersatz sein.

Bisher wird diese Gingiva-Restauration bzw. Maske auf einem Gipsmodell durch den Zahntechniker individuell gefertigt und danach vom Zahnarzt im Mund des Patienten angepasst. Bei großen Zahnfleischdefekten, z.B. infolge einer fortgeschrittenen Parodontitis, ist eine herausnehmbare Zahnfleischmaske oft die einzige Lösung, um die ursprüngliche Ästhetik wieder nachzubilden oder wiederherzustellen. Nach Abdrucknahme wird im zahntechnischen Labor aus Kunststoff eine hauchdünne zahnfleischfarbene Maske angefertigt, die den Zähnen ganz präzise anliegt. Der Tragekomfort dieser Zahnfleischmaske ist für den Patienten in der Regel sehr gut. Auch werden Ästhetik und Phonetik (Aussprache) entscheidend verbessert. Zur Zahnreinigung kann die Maske einfach herausgenommen und anschließend wiedereingesetzt werden.

Typ 2: Im Bereich der Zahntechnik wird ein weichbleibendes Material, das auf dem Gipsmodell das Zahnfleisch imitiert, ebenfalls Zahnfleischmaske genannt. Durch ein derartiges Hilfsmittel lassen sich die optischen, räumlichen und ästhetischen Verhältnisse im Mund sehr gut widerspiegeln. Nachteilig an den Zahnfleischmasken oder Gingiva-Restaurationen des Standes der Technik ist jedoch ihre monotone Farbgebung. Bislang werden die Gingiva-Restaurationen aus einem einzigen roten Farbton gefertigt, sodass es kein wirtschaftliches Verfahren zur Übertragung der natürlich auftretenden Gingiva-Rottöne gibt.

Die DE 8121341 U1 offenbart eine Zahnfleischmaske zur Herstellung und Einpassung des Zahnersatzes an die Zahnfleischpartie und damit ein Hilfsmittel für den Zahntechniker, um die Präparationsgrenze im Modell zu simulieren. Hergestellt wird die Zahnfleischmaske durch Einfüllen von fließfähigem, härtbarem Material in die Zwischenräume des Gipsmodells. Erhalten wird eine einfarbige Zahnfleischmaske.

DE 2744662 C3 offenbart eine Zahnfleischmaske und ein Verfahren zu deren Herstellung aus einem Silikonkautschuk, die nach Art des natürlichen Zahnfleisches strukturiert ist. Herstellbedingt wird eine Teigmasse eines Silicons in eine Pressform gegeben und somit eine einfarbige Zahnfleischmaske erhalten.

Ein wesentlicher ästhetischer Nachteil der Gingiva-Restaurationen des Standes der Technik ist die herstellbedingte Einfarbigkeit. Zudem gibt es bislang kein Verfahren zur Erfassung und Analyse der Gingiva-Situation bezüglich der verschiedenen Rottöne und deren Farbverläufe, da bislang lediglich eine Abdrucknahme der Gebiss-Situation erfolgte.

Eine Zuordnung von Farben im Dentalbereich ist bislang nur für die Zahnfarben bekannt.

Aufgabe der Erfindung war es, ein wirtschaftliches Verfahren zur Erfassung der Rottöne der Gingiva-Situation, deren Farbverläufen, sowie insbesondere in Kombination mit der Morphologie und Geometrie der Gingiva-Situation zu ermöglichen. Zur Durchführung des Verfahrens sollte zudem eine Vorrichtung und eine Software zur Lösung der Aufgabe aufgefunden werden. Ebenfalls bestand die Aufgabe eine sehr ästhetische und natürlich wirkende Gingiva für eine Teil- oder Totalprothese bereitzustellen.

Gelöst werden die Aufgaben entsprechend dem Gegenstand des Anspruchs 1, wobei vorteilhafte Ausgestaltungen in den Unteransprüchen und detailliert in der Beschreibung erläutert sind.

Es wird offenbart eine Gingiva-Indexierungsvorrichtung, die a) mindestens eine Farbregion aufweist, wobei die Farbregion mindestens einen roten Farbton aufweist, und b) der Indexvorrichtung mindestens eine Farbgraukarte und/oder eine Graukarte zugeordnet ist, insbesondere weist die Indexvorrichtung mindestens einen Bereich mit einer Farbgraukarte und/oder einer Graukarte auf.

Dabei kann die Farbgraukarte oder Graukarte beispielsweise der Basis oder dem Flächenelement der Indexvorrichtung entsprechen. Gleichfalls kann die Farbgraukarte und/oder Graukarte in einem Bereich analog einer Farbregion angeordnet sein. Ebenfalls Gegenstand der Erfindung ist ein Kit umfassend eine Gingiva-Indexierungsvorrichtung, die mindestens eine Farbregion aufweist, wobei die Farbregion mindestens einen roten Farbton bzw. Rotton aufweist wobei das Kit eine Farbgraukarte und/oder einer Graukarte umfasst.

Vorzugsweise weist eine Farbregion einen Rotton auf und ist insbesondere von weiteren Farbregionen der Indexierungsvorrichtung separiert. Alternativ kann eine Farbregion auch verschiedene definierte Rottöne in einer definierten Anordnung oder in einem typischen Farbverlauf aufweisen. Um ein hervorragendes Ergebnis bezüglich der Erfassung der Gingiva-Situation mithilfe der Indexierungsvorrichtung zu erreichen, können die Farbregionen bevorzugt dreidimensionale Farbkörper sein, die vorzugsweise typische rote Farbverläufe und/oder eine typische Morphologie eines Bereichs einer Gingiva wiedergeben.

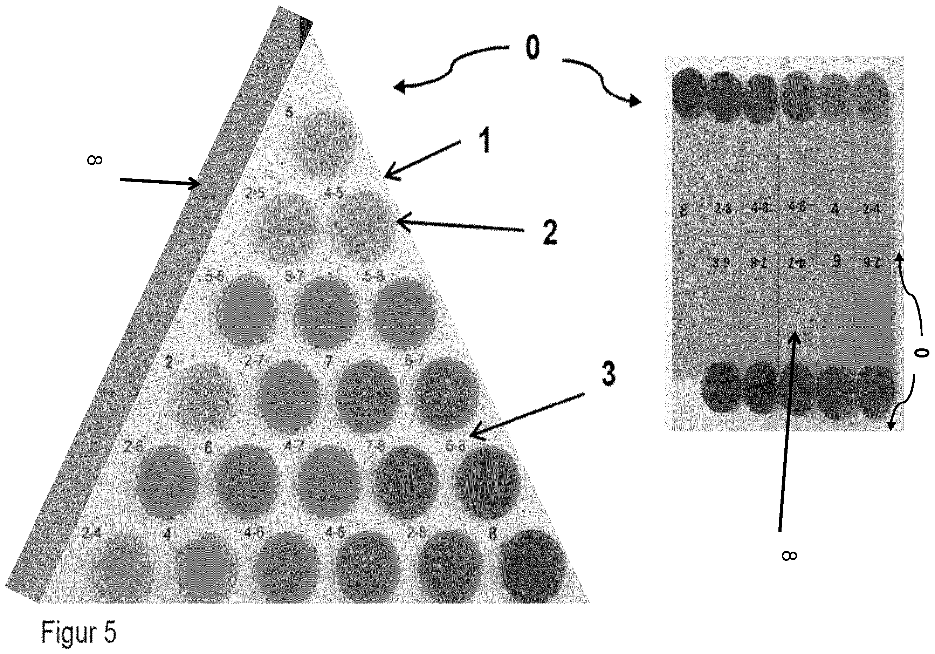

Es wird auch offenbart eine Indexierungsvorrichtung deren Farbregionen jeweils eine Farbmischanleitung zugeordnet ist. Die Farbmischanleitung ist beispielsweise der Indexvorrichtung in Figur 5 zu entnehmen dabei bedeutet 5-6, das die Farbe erhältlich ist, indem die dentalen Farben 5 und 6 im Gewichts- oder Volumenverhältnis 1:1 miteinander gemischt werden.

Die Farbgrau- und/oder Graukarte gemäß der Erfindung dient einer Bildbearbeitungssoftware dabei als Referenz zur Bestimmung der Farbtemperatur des Lichts, des Weißabgleichs und/oder zur Beurteilung einer Farbstichanpassung des Lichts. Erfindungsgemäß ist es daher nicht notwendig, dass die Farbgraukarte oder die Graukarte einer Karte entspricht, sondern dass die Farbgraukarte oder die Graukarte die Informationen trägt, die zur Kalibrierung oder Durchführung eines Weißabgleiches und/oder einer Farbstichbeurteilung oder Anpassung des Farbstichs benötigt werden.

Besonders bevorzugt ist jeder Farbregion der Indexierungsvorrichtung, vorzugsweise jeweils unabhängig, mindestens eine Kodierung zugeordnet. Über die Kodierung wird eine Farbmischanleitung zur farblichen Nachbildung der Gingiva bereitgestellt. Die Kodierung oder kodierten Farbregionen umfassen Kodierungen, wie Buchstabenkodes, Buchstaben- und Zahlenkodes, Zahlenkodes, Zeichenkodes, wie vorzugsweise Barcodes, Strichkodes, 2D- oder 3D-Barkodes, sowie weitere dem Fachmann bekannte Kodierungen. Die Kodierung kann auch als Farbindikator bezeichnet werden. Die Farbmischanleitung wird zur naturgetreuen Generierung der Farben der Gingiva-Restauration verwendet. Eine Gingiva-Restauration weist daher eine naturgetreue Nachbildung der Farben, Farbverläufe der Rottöne der natürlichen Gingiva auf, vorzugsweise mindestens 2 bis 5000 verschiedene Rottöne, besonders bevorzugt 5 bis 2000 verschiedene Rottöne.

Eine Gingiva-Indexierungsvorrichtung ist eine Vorrichtung, die es ermöglicht, Bereiche der Gingiva, insbesondere der menschlichen Gingiva, mithilfe von definierten roten Farbregionen zu kartographieren, also räumlich und geometrisch zu erfassen, zu analysieren und zu dokumentieren. Die Gingiva-Indexierungsvorrichtung erlaubt insbesondere eine reproduzierbare Erfassung, Analyse und/oder Dokumentation der Gingiva-Situation bezüglich der Rottöne, deren Farbverläufen und/oder der Morphologie der Gingiva-Situation. Die Gingiva-Indexierungsvorrichtung ermöglicht die topographische Zuordnung der Farben in der Restauration. Die Indexierungsvorrichtung und das erfindungsgemäße Verfahren erlauben die Erfassung der Zahnfleischfarben, der Zahnfleischcharakteristik.

Eine Gingiva-Indexierungsvorrichtung kann zur mehrfachen oder zur einmaligen Verwendung gedacht sein. Beispielweise kann eine Indexierungsvorrichtung als ein Abreißheft mit verschiedenen Streifen mit Farbregionen vorliegen. Ebenso ist ein Kit mit Farbkörpern denkbar, wobei die Farbkörper mit einem Instrument, bzw. einer Pinzette an die Gingiva gehalten werden. Generell kann eine Gingiva-Indexierungsvorrichtung jede sinnvolle Ausgestaltung aufweisen.

Eine Farbregion entspricht einem abgegrenzten zwei- oder dreidimensionalen Bereich umfassend mindestens einen Rotton.

Eine Gingiva-Restauration ist eine Nachbildung der fehlenden Zahnfleischpartie, der fehlenden Gingiva bzw. der fehlenden Gingiva-Partie, insbesondere für einen Bereich der Gingiva. Die Gingiva-Restauration kann vorteilhaft auch Teil einer prothetischen Versorgung mit Zahnersatz sein.

Klassierte Farbkörper sind Farbkörper, insbesondere dreidimensionale Farbkörper, mit mindestens einer kodierten Farbregion. Die Gingiva-Indexierungsvorrichtung mit umfassend klassierten Farbkörpern kann beispielsweise eine Box mit separierten Fächern umfassen, in denen in den jeweiligen Fächern je eine Vielzahl je einer Art der klassierten Farbkörper bereitgestellt werden. Die klassierten Farbkörper sind vorteilhaft zur einmaligen Verwendung vorgesehen.

Gegenstand der Erfindung ist auch eine Indexierungsvorrichtung umfassend 1 bis 5000 Farbregionen, wobei jede Farbregion unabhängig voneinander einen definierten voneinander abweichenden roten Farbton aufweist. Insbesondere 2 bis 2000 Farbregionen, vorteilhaft umfassend 2 bis 100 Farbregionen, bevorzugt mit 5 bis 100 Farbregionen, besonders bevorzugt mit 10 bis 100 Farbregionen, weiter bevorzugt sind 6-15 oder auch 21 Farbregionen, insbesondere mit mindestens einem Farbton, vorzugsweise umfasst jede Farbregion einen Rotton, der von den Rottönen der weiteren Farbregionen abweicht. Besonders bevorzugt umfasst eine Farbregion einen typischen Farbverlauf von Rottönen eines Bereiches der Gingiva.

Entsprechend einer bevorzugten Alternative umfasst die Indexierungsvorrichtung mindestens eine Farbregion, insbesondere mindestens zwei bis 2000 Farbregionen, die jeweils farbige Körper sind. Vorteilhaft ist jede Farbregion jeweils unabhängig ein farbiger Körper, bevorzugt ist der farbige Körper von der Indexierungsvorrichtung lösbar. Ein farbiger Körper kann Polymere, keramische Materialien, Kompositmaterialien, Gläser oder Hybridmaterialien umfassen.

Ferner ist es bevorzugt, wenn die Farbregionen in einer definierten Anordnung zueinander an der Indexierungsvorrichtung positioniert sind. Im Sinne der Erfindung umfasst eine Indexierungsvorrichtung Farbregionen, die auf, in, an, festverbunden oder lösbar, abtrennbar etc. mit der Vorrichtung verbunden oder an ihr angebracht sind. Dabei ist es bevorzugt, wenn die Farbregionen der Indexierungsvorrichtung von einem hellen Rotton bis hin zu einem dunklen Rotton und/oder von einem Rot mit Gelbanteilen zu einem Rot mit Blauanteilen angeordnet sind. Vorteilhafte Farbverläufe umfassen eine Kodak-Farbskala, RGB-Modell, CMYK-Modell, Pantone, HKS-Fächer, RAL-Farbsystem oder andere dem Fachmann geläufige Farbsysteme, die Rottöne oder Rotnuancen umfassen.

Vorteilhafte Indexierungsvorrichtungen weisen Farbregionen auf, die jeweils farbige Körper sind und vorteilhaft auf einem Element angeordnet sind. Als Elemente eignen sich alle zwei oder dreidimensionalen Elemente, wie beispielsweise Flächenelemente, längliche dreidimensionale Körper, Stäbchen, Röhren, Folien, Instrumente, dreidimensionale Elemente. Dabei können die Flächenelemente vorteilhaft aus flexiblem Material oder starrem Material gebildet werden. Vorteilhaft sind die Farbregionen, insbesondere die farbigen Körper lösbar oder abtrennbar an dem Element angebracht. Das Element als solches ist vorzugsweise aufteilbar, wie beispielsweise schneidbar, zerreißbar, auseinanderziehbar etc. Die Farbregionen oder Elemente können auch auf der Gingiva haften, um die Analyse zu vereinfachen. Somit umfasst die Erfindung auch verklebbare Farbregionen oder Elemente.

Vorteilhafte Flächenelemente können dreieckig, rechteckig, als Streifen; quadratische, kreisförmige, ovale Elemente vorliegen. Ebenso kann ein Element als Polygon, wie ein Pentagon, Hexagon, Heptagon, Oktagon etc. vorliegen und weist mindestens eine Farbregion auf, die an dem Element angeordnet ist, wobei verschiedene Farbregionen auf verschiedenen Elementen oder auf einem Element angeordnet sind.

Ebenso können die farbigen Elemente, insbesondere Körper, jede dreidimensionale Form aufweisen, wie beispielsweise, aber nicht abschließend, Polyeder, oval, stabförmig, Ausschnitt aus einer Kugeloberfläche.

Entsprechend einer besonders bevorzugten Ausführungsform sind
a) die Farbregionen, insbesondere die farbigen Körper, auf einem im Wesentlichen dreieckigen Flächenelement angeordnet, wobei sich der Farbverlauf des roten Farbtons entlang eines Schenkels des Dreiecks von einem Rot mit Gelbanteilen (rotgelb) zu einem Rot mit Blauanteilen (rot-blau) verändert und/oder sich von einem hellen roten Farbton zu einem dunklen roten Farbton verändert, insbesondere werden der Farbton rot-blau und der Farbton dunkel-rot als Farbtöne in einer Farbregion dargestellt, oder
b) die Indexierungsvorrichtung ist ein Flächenelement, insbesondere ein quadratisches oder rechteckiges Flächenelement, umfassend lösbare Farbkörper, abtrennbare Farbkörper oder lösbare Halterungen mit jeweils mindestens einem Farbkörper, lösbare Halterungen mit mindestens einer Farbregion je Halterung, abtrennbare Bereiche mit mindestens einer Farbregion oder abtrennbare Bereiche mit mindestens einem Farbkörper, wobei die Halterungen oder Bereiche vorteilhaft gebildet werden durch eine Perforation des Flächenelementes, insbesondere abtrennbare Streifen mit jeweils mindestens einem Farbkörper oder abtrennbare Streifen mit jeweils mindestens einer Farbregion, wobei vorzugsweise das Flächenelement teilbar ist, oder
c) die Indexierungsvorrichtung Teil eines Kits ist, umfassend Farbkörper, insbesondere klassierte Farbkörper, insbesondere Farbkörper deren jeweiliger roter Farbton eine Nuance von gelb nach blau und von hell rot bis dunkel rot aufweist, wobei die Farbkörper auf oder an einem Element anbringbar sind, insbesondere ist den Farbkörpern jeweils mindestens eine Kodierung zugeordnet. Vorteilhaft kann die Kodierung auf die Farbkörper aufgebracht sein. Die Farbkörper nach c) sind beispielsweise auf ein zahntechnisches Instrument anbringbar, so kann beispielsweise ein Instrument in den Farbkörper reingedrückt werden und der Farbkörper aufgespießt werden oder mit einer Pinzette an die Gingiva angehalten werden. Abtrennbar ist im Sinne von abschneiden, abreißen etc. zu verstehen. Alternativ können die Farbregionen oder Farbkörper wiederverklebbar sein.

Nach einer Alternative ist die Indexierungsvorrichtung eine einmal zu verwendende Indexierungsvorrichtung. Vorteilhaft ist die Vorrichtung biologisch abbaubar.

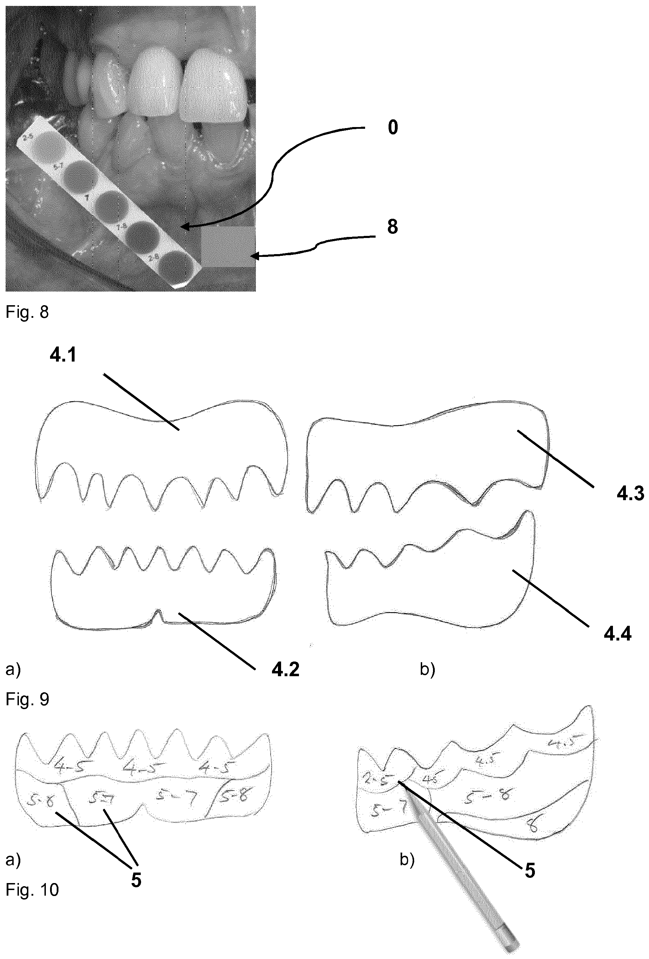

Gleichfalls Gegenstand der Erfindung ist ein Kit umfassend eine Gingiva Indexierungsvorrichtung und eine beschriftbare Gingiva-Vorlage. Unter einer beschriftbaren Gingiva-Vorlage ist eine Vorlage entsprechend den Figuren 9 a) und b) sowie den Figuren 10 a) und b) zu verstehen. Die Figuren 10 a) und b) stellen beschriftete Gingiva-Vorlagen dar. Alternativ kann die beschriftbare Gingiva-Vorlage auch ein dreidimensionales Modell der Gingiva-Situation oder des Gebisses oder eines Teils dieser Modelle sein. Unter einer beschriftbaren Gingiva-Vorlage ist ebenso ein virtuelles Motiv der Gingiva, das in einer Software dargestellt ist und in das die Kodierung eingefügt werden kann, zu verstehen. Die Gingiva-Vorlage kann ebenso ein zwei- oder dreidimensionales virtuelles Modell sein.

Gegenstand des Kits kann auch ein weichbleibendes Dental-Material, ein Komposit-Material oder ein keramisches Dentalmaterial zur Herstellung der Gingiva-Restauration sein.

Gegenstand der Erfindung ist ein Verfahren zur Erfassung der Gingiva-Situation, und zur Herstellung einer Gingiva-Restauration, indem
A) eine Analyse mindestens eines Bereiches der Gingiva-Situation, insbesondere der verbliebenen Gingiva, erfolgt in Bezug auf ihre roten Farbtöne, die Position der roten Farbtöne und/oder den Farbverlauf der roten Farbtöne und optional der Geometrie, insbesondere unter Berücksichtigung der Zahnsituation, und/oder der Morphologie, wobei die Analyse digital erfolgt, und
B) Erhalten von Gingiva-Informationen, insbesondere Erhalten von Gingiva-Informationen in Form einer Gingiva-Kartierung, vorteilhaft erfolgt die Analyse der Gingiva-Situation in Bezug auf mindestens 2, 3, 4, 5, 6, 7 bis zu 2000 Farbtöne, insbesondere werden digitale Gingiva-Informationen erhalten, bevorzugt werden die Gingiva-Informationen als digitaler Datensatz erhalten, vorzugsweise als erster digitaler-Datensatz, und
C)Herstellen eines virtuellen oder realen Modells einer Gingiva-Restauration, d.h. einer Nachbildung der fehlenden Gingiva-Partie, und insbesondere Herstellen einer Gingiva-Restauration, insbesondere der fehlenden Gingiva-Partie.

Verfahren zur Erfassung der Gingiva-Situation, insbesondere zur Erstellung einer virtuellen oder realen Gingiva-Restauration, in dem
A) eine Analyse mindestens eines Bereiches der Gingiva-Situation erfolgt in Bezug auf ihre roten Farbtöne, die Position der roten Farbtöne und/oder den Farbverlauf der roten Farbtöne und optional der Geometrie und/oder der Morphologie,
   wobei die A) Analyse digital erfolgt, und
   a.1) wobei mindestens ein Bereich der Gingiva-Situation zusammen mit einer Farbgrau- oder Graukarte und optional zusammen mit mindestens einem roten Farbton einer Indexierungsvorrichtung erfasst wird, insbesondere erfolgt die Erfassung mittels Photographieren oder Scannen zusammen mit einer Farbgrau- oder Graukarte,
   a.2) wobei digitale Gingiva-Informationen als digitaler Datensatz erhalten werden, insbesondere als erster digitaler Datensatz, und optional Bereitstellen, Speichern und/oder Versenden des Datensatzes,
   a.3) Durchführen eines Weißabgleichs und/oder einer Farbstichanpassung am digitalen Datensatz, insbesondere mit einer Software, wie einer Bildbearbeitungssoftware, und Erhalten eines farbangepassten digitalen Datensatzes (kalibrierter Datensatz),
   a.4) Analyse des Datensatzes auf Farbtöne und/oder Farbverläufe und
B) Erhalten von Gingiva-Informationen, und optional
   b.1) Zuordnen der Gingiva-Informationen zu vorkonfektionierten, insbesondere kodierten, dentalen Farben und/oder einer Farbmischanleitung dentaler Farben, und/oder
   b.2) Erstellen des digitalen Datensatz mit der Geometrie des virtuellen oder realen Gingiva-Modells der Gingiva-Restauration, optional unter Verwendung des farbangepassten digitalen Datensatz mit Gingiva-Informationen, und optional der Zahnsituation und/oder des Bereiches der Gesichtspartie, und optional
C) Herstellen eines virtuellen oder realen Modells einer Gingiva-Restauration, d.h. einer Nachbildung der fehlenden Gingiva-Partie.

Herstellen einer Gingiva-Restauration, wobei die Reihenfolge der Schritte a.3, b.1 und b.2 abweichen kann, beispielsweise mit der Reihenfolge der Schritte b.2, a.3, a.4, b.1 oder jeder beliebigen sinnvollen Reihenfolge. Sinnvoll kann auch die Durchführung des Schrittes b.2, in dem zunächst der Datensatz umfassend die Geometrie des virtuellen oder realen Gingiva-Modells bereitgestellt wird, vor den Schritten a.1, a.2, a.4 und /oder a.3 sein, so dass der farbangepasste Datensatz den geometrischen Daten vor Schritt b.1 hinzugefügt wird.

Dabei werden vorteilhaft die erfassten roten Farbtöne, die Positionen der roten Farbtöne, sowie die Position der roten Farbtöne zueinander, die Geometrie der Gingiva, wie auch die Zahnsituation und/oder die Morphologie der Gingiva, insbesondere der verbliebenen Gingiva, in mindestens zwei verschiedenen Bereichen der Gingiva-Situation anhand der in a) erhaltenden Gingiva-Informationen zur Herstellung der Gingiva-Restauration verwendet und/oder übertragen.

Beispielsweise werden die Gingiva-Informationen auf einen Abdruck der Gebiss-Situation, ein Gips-Modell der Gebisssituation oder eine beschriftbare Gingiva-Vorlage übertragen.

Bevorzugt werden mindestens 2 bis 5000 Rottöne, insbesondere 5 bis 30 Rottöne (Farbtöne), besonders bevorzugt mindestens 10 bis 25 Rottöne erfasst, sowie vorteilhaft die der Interdentalpapille (Papilla interdentalis).

Die Gingiva-Informationen können vorteilhaft mit einer Auflösung von 50 dpi erhalten werden, vorzugsweise 150 dpi, insbesondere größer gleich 300 dpi, bevorzugt mit einer guten Auflösung von größer gleich 600 dpi (Bildpunkte per inch) bis zu einer hohen Auflösung von größer gleich 1200 dpi oder höher. Vorteilhaft können die Gingiva-Informationen zusätzlich oder alternativ mit einer Detailgenauigkeit von kleiner gleich 100 µm, insbesondere kleiner gleich 50 µm, besonders bevorzugt bis kleiner gleich 30 µm, bis kleiner gleich 20 erfasst und vorzugsweise reproduziert werden.

Das dreiecksförmige Zahnfleisch zwischen den Zähnen wird als "Interdentalpapille" (Papilla interdentalis), die Grenzlinie zwischen dem Zahnfleisch und der verschiebbaren dunkelroten Mundschleimhaut als Mukogingivallinie oder mukogingivale Grenze bezeichnet. Diese Bereiche werden vorteilhaft besonders detailliert analysiert und die entsprechenden Gingiva-Informationen festgehalten.

Ferner ist Gegenstand der Erfindung ein Verfahren, in dem die A) Analyse digital erfolgt,
- indem der mindestens eine Bereich der Gingiva-Situation, insbesondere der verbliebenen Gingiva, und optional eines Bereiches der Gesichtspartie zwei- oder dreidimensional digital erfasst wird, insbesondere wird der Bereich gescannt oder photographiert vorzugsweise zusammen mit einer Farbgrau- und/oder einer Graukarte zur Kalibrierung, wobei digitale Gingiva-Informationen als digitaler Datensatz erhalten werden, insbesondere als erster digitaler Datensatz, vorzugsweise werden Farbinformationen erhalten sowie optional zusätzlich dreidimensionale Informationen, oder
- indem eine zwei- oder dreidimensionale Erfassung mindestens eines Bereiches der Gingiva- und der Zahnsituation und optional eines Bereiches der Gesichtspartie erfolgt und mindestens eines Teils einer Farbregion mit rotem Farbton der Indexierungsvorrichtung, wobei die Erfassung zusammen mit der Erfassung mindestens eines Teils einer Farbgrau- und/oder Graukarte zur Kalibrierung erfolgt und digitale Gingiva-Informationen als digitaler Datensatz erhalten werden insbesondere als erster digitaler Datensatz.

Alternativ erfolgt mindestens eine Erfassung eines Bereiches der Gingiva-Situation und der Zahnsituation und einer roten Farbregion und optional eines Bereiches der Gesichtspartie, zusammen mit mindestens einem Teil einer Farbgrau- und/oder Graukarte, insbesondere erfolgt die Erfassung durch Scannen oder Photographieren, wobei digitale Gingiva-Informationen als digitaler Datensatz erhalten werden.

Vorzugsweise erfolgt eine zwei- oder dreidimensionale Erfassung mittels Intraoralscan oder mittels Photographieren. Nach dieser Alternative wird ein Verfahren umfasst, indem die Farbregionen der Indexvorrichtung an die Gingiva gehalten werden und die Zuordnung mittels eines Photos oder Intraoralscans erfasst wird. Zur späteren Kalibrierung der Farbtöne im Datensatz wird die Farb- oder Graukarte miterfasst.

Die so erhaltenen digitalen Daten können dann vom Zahnarzt leicht an den Zahntechniker zur Herstellung der Gingiva-Restauration übermittelt werden. Ein besonderer Vorteil des erfindungsgemäßen Verfahrens ist die Möglichkeit der Erstellung eines virtuellen Modells der Gingiva-Restauration und Übermittlung an den Zahnarzt und den Patienten bevor ein reales Gingiva-Modell oder die Gingiva-Restauration erstellt wird. So ist es möglich, mit dem Zahnarzt und dem Patienten die Ästhetik frühzeitig abzustimmen.

Die Analyse der erfassten Gingiva-Informationen, insbesondere als digitaler Datensatz, erfolgt über eine manuelle oder automatische Analyse und Zuordnung zu Farbregionen einer Indexvorrichtung oder einer beschriftbaren Gingiva-Vorlage.

Gleichfalls Gegenstand der Erfindung ist ein Verfahren, indem die A) Analyse digital erfolgt, 1) indem mindestens ein Bereich der Gingiva-Situation und optional ein Bereich der Gesichtspartie unter Verwendung einer Farbgraukarte und/oder einer Graukarte zur Kalibrierung erfasst wird, wobei der Bereich der Gingiva zusammen mit der Karte zwei- oder dreidimensional digital erfasst wird, indem der Bereich und zumindest ein Teil der Karte gescannt oder photographiert wird, wobei digitale Gingiva-Informationen als digitaler Datensatz erhalten werden, oder 2) indem eine zwei- oder dreidimensionale Erfassung mindestens eines Bereiches der Gingiva und optional der Zahnsituation und optional eines Bereiches der Gesichtspartie erfolgt und mindestens eine Farbregion mit einem roten Farbton einer Indexierungs-vorrichtung, wobei die Erfassung zusammen mit der Erfassung mindestens eines Teils einer Farbgraukarte und/oder eine Graukarte zur Kalibrierung erfolgt, insbesondere erfolgt die Erfassung durch Scannen oder Photographieren, wobei digitale Gingiva-Informationen als digitaler Datensatz erhalten werden.

Alternativ kann auch eine Abformmasse oder ein Modell der Gingiva digital dreidimensional erfasst werden, und eine digitaler Datensatz mit Geometriedaten erhalten wird. Anschließend kann dieser Datensatz mit den digitalen Gingiva-Informationen versehen werden.

Die digitalen Daten können gespeichert, übertragen sowie modifiziert werden, um ein Modell einer virtuellen Gingiva-Restauration herzustellen.

Alternativ oder zusätzlich kann die Analyse (ii) manuell erfolgen indem 3) manuell eine zwei- oder dreidimensionale Erfassung mindestens eines Bereiches der Gingiva erfolgt oder mindestens eine Erfassung eines Bereiches der Gingiva und der Zahnsituation oder mindestens eine Erfassung eines Bereiches der Gingiva und einer Farbregion einer Indexierungsvorrichtung oder mindestens eine Erfassung eines Bereiches der Gingiva und der Zahnsituation und einer Farbregion erfolgt, und manuelle Gingiva-Informationen erhalten werden. Nach dieser Variante können mittels Indexvorrichtung ermittelte Gingiva-Informationen als schriftliche Notizen beispielsweise auf der Gingiva-Vorlage notiert werden.

Ebenfalls Gegenstand der Erfindung ist ein Verfahren, in dem die Gingiva analysiert wird: a) manuell unter Anwendung einer Indexierungsvorrichtung, indem die Gingiva mit der Indexierungsvorrichtung auf Farbtöne und/oder Farbverläufe untersucht wird, insbesondere im Bereich der Gingiva im Frontzahnbereich (anterior) und/oder im Seitenzahnbereich (posterior) auf Farbtöne und/oder Farbverläufe, insbesondere mandibulär und/oder maxibulär, besonders bevorzugt wird die Gingiva analysiert auf (i) Farbverläufe der Papillen, krestal, im Bereich des Sulcus, zusätzlich oder alternativ wird die (ii) Geometrie der Gingiva analysiert, insbesondere in Bezug auf die Position der Papillen, der krestalen Situation, der Zahnbreite, Alveolarkamm (Kieferkamm), und/oder die Zahnsituation, wie die Sichtbarkeit der Zahnhälse und/oder es wird die (iii) Morphologie der Gingiva analysiert, und Erhalten von Gingiva-Informationen, insbesondere als manuelle Gingiva-Informationen.

Bevorzugt ist ein Verfahren in dem die Analyse (i) digital unter Anwendung einer Software erfolgt, indem anhand einer Software ein Weißabgleich und/oder ein Abgleich des Farbstichs, bspw. Farbstichbeurteilung, in Korrelation zur Farbgraukarte oder Graukarte am digitalen Datensatz der digitalen Gingiva-Informationen erfolgt, und ein farbangepasster digitaler Datensatz erhalten wird.

Dieser Datensatz ist ein kalibrierter Datensatz. Der farbangepasste digitale Datensatz der Gingiva und optional der Zahnsituation wird manuell mit einem Cursor oder automatisiert auf Farbtöne und/oder Farbverläufe analysiert, besonders bevorzugt wird die Gingiva analysiert auf (i) Farbverläufe der Papillen, krestal, im Bereich des Sulcus, zusätzlich oder alternativ wird die (ii) Geometrie der Gingiva analysiert, insbesondere in Bezug auf die Position der Papillen, der krestalen Situation, der Zahnbreite, Alveolarkamm (Kieferkamm), und/oder die Zahnsituation, wie die Sichtbarkeit der Zahnhälse und/oder es wird die (iii) Morphologie der Gingiva analysiert. Dabei werden Gingiva-Informationen erhalten, und optional werden den Gingiva-Informationen und/oder den Gingiva-Bereichen jeweils unabhängig eine oder mehrere definierte, konfektionierte, dentale Farben, insbesondere kodierte Farben, und/oder eine oder mehrere Farbmischanleitungen dentaler Farben zugeordnet, insbesondere von denen manuell oder automatisiert eine dentale Farbe oder eine Farbmischanleitung dentaler Farben ausgewählt werden kann.

Erfindungsgemäß bevorzugt wird die Gingiva digital und optional manuell analysiert: b) digital und optional manuell unter Anwendung einer Software, indem die Gingiva-Situation und optional die Zahnsituation mit der Software auf Farbtöne und/oder Farbverläufe untersucht wird, insbesondere im Bereich der Gingiva im Frontzahnbereich (anterior) und/oder im Seitenzahnbereich (posterior) auf Farbtöne und/oder Farbverläufe, insbesondere mandibulär und/oder maxibulär, besonders bevorzugt wird die Gingiva analysiert auf (i) Farbverläufe der Papillen, krestal, im Bereich des Sulcus, zusätzlich oder alternativ wird die (ii) Geometrie der Gingiva analysiert, insbesondere in Bezug auf die Position der Papillen, der krestalen Situation, der Zahnbreite, Alveolarkamm (Kieferkamm), und/oder die Zahnsituation, wie die Sichtbarkeit der Zahnhälse und/oder es wird die (iii) Morphologie der Gingiva analysiert, und Erhalten von Gingiva-Informationen, insbesondere als manuelle Gingiva-Informationen. Vorzugsweise werden die Gingiva-Informationen in eine virtuelle Gingiva-Vorlage übertragen, vorzugsweise automatisiert durch die Verwendung einer Software.

Besonders bevorzugt werden die Gingiva-Informationen in Form einer Gingiva-Kartierung erhalten, insbesondere als digitaler Datensatz. Vorteilhaft kann aus diesen Gingiva-Informationen ein virtuelles und/oder reales Modell einer Gingiva-Restauration unter Berücksichtigung des Ausgleiches des Zahnfleischrückgangs und/oder des Kieferschwundes erstellt werden. Besonders bevorzugt ist eine Gingiva-Kartierung, insbesondere mit einem Motiv der Gingiva, mit zugeordneten, definierten, vorkonfektionierten, kodierten, dentalen Farben oder Farbmischanleitungen für dentale Farben. Als dentale Farben gelten Zusammensetzungen dentaler Farben zur Herstellung einer Gingiva-Restauration.

Entsprechend einer besonders bevorzugten Alternative werden die digitalen Gingiva-Informationen des digitalen Datensatzes a) digital kodierten Farbregionen zugeordnet, oder b) manuell kodierten Farbregionen der Indexvorrichtung zugeordnet, wobei jeweils unabhängig der Kodierung bzw. den kodierten Farbregionen eine Farbmischanleitung zugeordnet ist. Über die Farbmischanleitung ist eine naturgetreue farbliche Wiedergabe der Gingiva auf eine Gingiva-Restauration möglich.

Ebenso ist Gegenstand der Erfindung ein Verfahren zur Herstellung einer virtuellen oder realen Gingiva-Restauration, indem (i) in einem Schritt vor der Erstellung des virtuellen oder realen Gingiva-Modells ein erster digitaler Datensatz umfassend digitale Gingiva-Informationen eines ersten virtuellen Modells der Gingiva-Situation und optional der Zahnsituation und/oder einem Bereich der Gesichtspartie, insbesondere zusammen mit der Erfassung einer Farbgraukarte und/oder einer Graukarte zur Kalibrierung und optional mindestens eine Farbregion mit einem roten Farbton einer Indexierungsvorrichtung erstellt wird, vorzugsweise mittels Intraoralscan oder mindestens eines einzelnen Photos, bspw. mit einem Smartphone. Der erste digitale Datensatz entspricht vorzugsweise dem Datensatz mit digitalen Gingiva-Informationen.

Alternativ kann ein 3D-Scan eines Silikonabdrucks oder ein 3D-Scan eines realen Modells erfolgen, insbesondere eines Gipsmodels, und mit digitalen Gingiva-Informationen (Farbinformationen der Gingiva) ergänzt werden.

Optional erfolgt ein (i.0) Bereitstellen, Speichern, Versenden des jeweiligen Datensatzes. Ferner kann das Verfahren die folgenden Schritte umfassen
(i.1) Durchführen eines Weißabgleichs und oder eines Abgleichs des Farbstichs und Erhalten eines farbangepassten digitalen Datensatzes, und optional (i.2) Analyse des farbangepassten Datensatzes auf Farbtöne und/oder Farbverläufe und Erhalten von Gingiva-Informationen, und optional (i.3) Zuordnen der Gingiva-Informationen zu vorkonfektionierten, insbesondere kodierten, dentalen Farben und/oder einer Farbmischanleitung dentaler Farben, und optional (ii) in einem nächsten Schritt der digitale Datensatz mit der Geometrie des virtuellen oder realen Gingiva-Modells der Gingiva-Restauration erstellt wird und optional unter Verwendung des farbangepassten digitalen Datensatz oder enthaltend die Informationen aus dem farbangepassten Datensatz, und
(iii) optional Herstellen einer Gingiva-Restauration. Vorzugsweise wird die Gingiva-Restauration unter Ausgleich des Zahnfleischrückgangs, Kieferschwundes und der vorgenannten roten Farbverläufen sowie optional der Morphologie hergestellt. Dabei kann die Reihenfolge von (i.1 bis i.3) und (ii) umgekehrt sein, indem zunächst (ii) und anschließend (i.1 bis i.3) durchgeführt werden.

Die Herstellung erfolgt vorzugsweise unter Verwendung definierter vorkonfektionierter, kodierter Farben, und/oder Verwendung der Farbmischanleitung oder unter Verwendung der Gingiva-Kartierung, insbesondere mit definierten vorkonfektionierten, kodierten Farben oder Farbmischanleitungen.

Offenbart ist auch ein Verfahren zur Herstellung einer Gingiva-Restauration sowie eine Gingiva-Restauration, erhältlich nach diesem Verfahren, indem eine Erfassung der Gingiva-Situation nach dem erfindungsgemäßen Verfahren erfolgt und die Herstellung eines virtuellen oder realen Modells einer Gingiva-Restauration erfolgt, indem die roten Farbtöne, die Position der roten Farbtöne, die Geometrie der Gingiva und/oder die Morphologie der Gingiva in mindestens zwei verschiedenen Bereichen der Gingiva anhand der erhaltenden Gingiva-Informationen zur Herstellung der Gingiva-Restauration verwendet bzw. übertragen werden.

Zur Herstellung der Gingiva-Restauration können die Gingiva-Informationen der erfassten Gingiva-Situation verwendet werden und unter Berücksichtigung und Ausgleich des Zahnfleischrück- und/oder des Kieferrückgangs sowie unter ästhetischen Gesichtspunkten ein virtuelles oder reales Modell einer Gingiva-Restauration simuliert und/oder erstellt werden. Das virtuelle Modell kann vorzugsweise mit dem Patienten vor einer Anfertigung abgestimmt werden. Vorteilhaft kann das virtuelle Modell in die Gingiva-Situation einkopiert werden und mit dem Patienten erläutert werden.

Offenbart ist auch eine Gingiva-Restauration erhältlich nach dem erfindungsgemäßen Verfahren. Vorteilhafte Gingiva-Restaurationen werden aus einem weichbleibendem Dental-Material, einem Komposit-Material oder einem keramischen Dentalmaterial gefertigt.

Hergestellt werden können die Gingiva-Restaurationen beispielsweise nach einem Verfahren entsprechend der Offenbarung der DE 10 2012 012 346 A1 indem während des Druckvorgangs die Gingiva-Farbtöne entsprechend der Farbmischanleitung eingestellt werden.

Offenbart ist auch ein digitaler Datensatz, erhältlich nach dem erfindungsgemäßen Verfahren, der insbesondere auf einem Datenträger gespeichert ist.

Zusätzlich ist offenbart eine Software zur Verarbeitung digitaler Datensätze, erhalten nach dem erfindungsgemäßen Verfahren, insbesondere auf einem computerlesbaren Medium, wie einem Datenträger oder einem Computer, insbesondere zur Verarbeitung digitaler Datensätze umfassend Gingiva-Informationen, vorzugsweise zur Analyse mindestens eines Bereiches der Gingiva, insbesondere der Gingiva-Situation, in Bezug auf ihre roten Farbtöne, die Position der roten Farbtöne, den Farbverlauf der roten Farbtöne, Geometrie und/oder die Morphologie und zur Bereitstellung von Gingiva-Informationen und optional zur Zuordnung kodierter Farbregionen, wobei der Kodierung eine Farbmischanleitung zugeordnet ist und/oder zur Erstellung digitaler Datensätze der Gingiva-Restauration.

Vorteilhaft erlaubt die Software die Analyse von digital zur Verfügung gestellten Daten des mindestens einen Bereiches der Gingiva, insbesondere der verbliebenen Gingiva bzw. Gingiva-Situation, in Bezug auf ihre roten Farbtöne, die Position der roten Farbtöne, den Farbverlauf der roten Farbtöne, Farbnuancen, Geometrie, Zahnsituation und/oder die Morphologie, und stellt vorzugsweise Gingiva-Informationen als digitalen Datensatz zur Verfügung. Optional erlaubt die Software die Zuordnung der verschiedenen Bereiche der Gingiva-Situation zu kodierten Farbregionen, wobei der Kodierung eine Farbmischanleitung zugeordnet ist. Alternativ oder zusätzlich erlaubt die Software die Berechnung digitaler Datensätze einer virtuellen Gingiva-Restauration oder schlägt virtuelle Modelle einer Gingiva-Restauration vor.

Die Software kann in Form einer Applikation (App, mobile App) auf einem Computer, insbesondere auf einem mobilen Computer, insbesondere auf einem Smartphone, einer Digitalkamera oder jedem anderen geeigneten beliebigen elektronischen Gerät bereitgestellt werden.

Zur naturgetreuen Nachbildung und Herstellung von Gingiva-Restaurationen können diese über den digitalen Datensatz mittels Fräsen aus einem Körper und anschließendem Aufdrucken der farbigen Gingiva, mittels Rapid-Prototyping, insbesondere mittels Licht härtender generativer Verfahren schichtweise aus einer härtbaren Zusammensetzung von Monomeren und/oder Prepolymeren, hergestellt werden, vorzugsweise einer Strahlen-härtbaren Zusammensetzung. Ebenso sind Stereolithographie, DLP-Verfahren oder 3D-Druckverfahren anwendbar. Alternativ ist die manuelle, klassische Herstellung einer Gingiva-Restauration unter Berücksichtigung der Gingiva-Informationen, zur Nachbildung der roten Ästhetik, möglich.

Die Erfindung wird anhand der nachfolgenden Figuren eingehender erläutert, ohne die Erfindung auf die Beispiele zu beschränken.

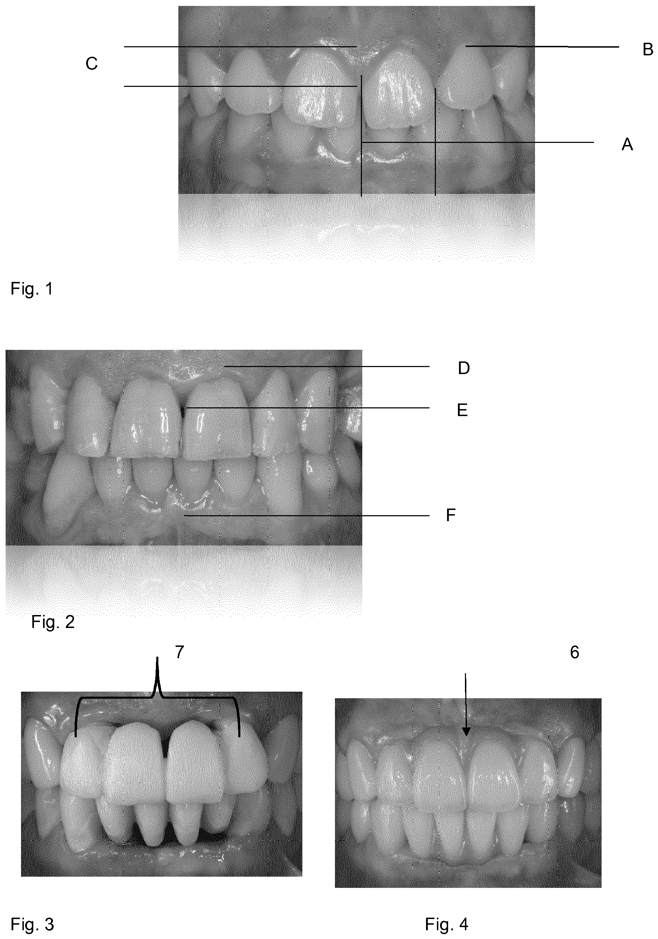

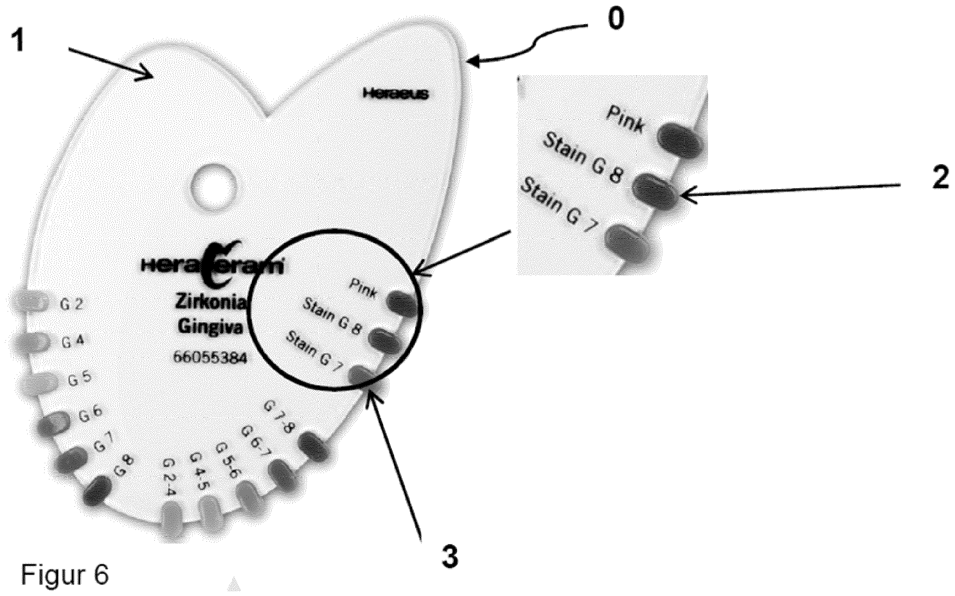

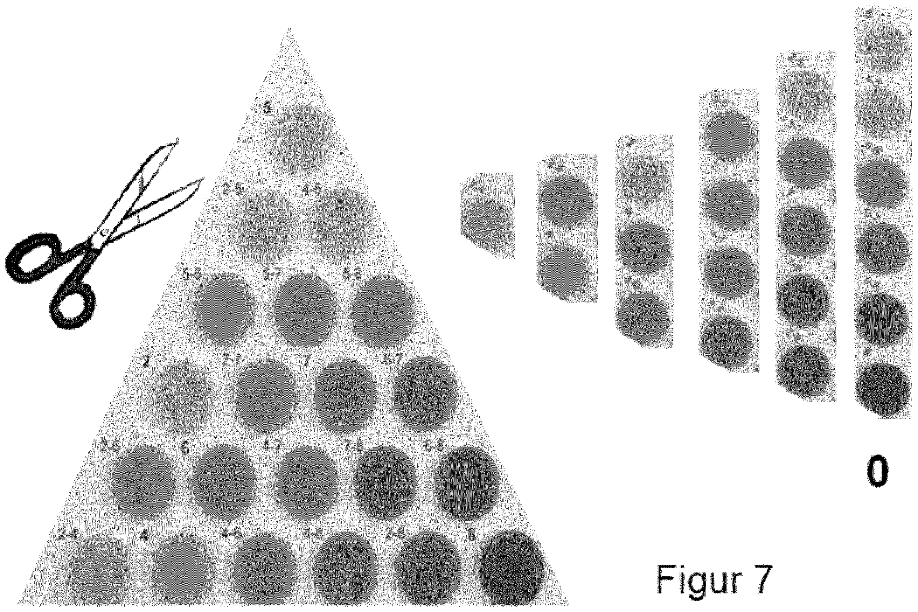

Figuren 1 und 2 zeigen einen Teil der zu analysierenden Bereiche zur Feststellung der Gingiva-Situation. Figur 3 zeigt eine Gingiva-Situation mit Gingiva-Defizit 7, während Figur 4 die Mundpartie mit Gingiva-Restauration darstellt. Die Figuren 5, 6 und 7 zeigen eine Gingiva-Indexierungsvorrichtung 0 in Form eines Elementes, insbesondere eines Flächenelements 1 mit einer Vielzahl an Farbregionen 2, die in diesen Alternativen als farbige Körper mit einem definierten Rotton ausgebildet sind. Die Farbregionen 2 sind kodiert 3, wobei der Kodierung eine Farbmischanleitung (manuell oder digital) zugeordnet ist, so dass über die erfasste Farbregion und deren Kodierung die naturgetreue Nachbildung der verschiedenen Gingiva-Rottöne, deren Farbverläufen, Morphologie und Geometrie möglich ist. Die Nachbildung kann über Drucken, Mischen etc. von Kunststoffen, gefüllten Kunststoffen, elastomeren Kunststoffen, Keramiken oder Kompositen erfolgen. Figur 7 stellt eine Indexierungs-vorrichtung dar und eine Möglichkeit der Abtrennung der Farbregionen durch Zerschneiden der Vorrichtung. Die dargestellte Indexierungsvorrichtung ist eine Single-Use Indexierungsvorrichtung.

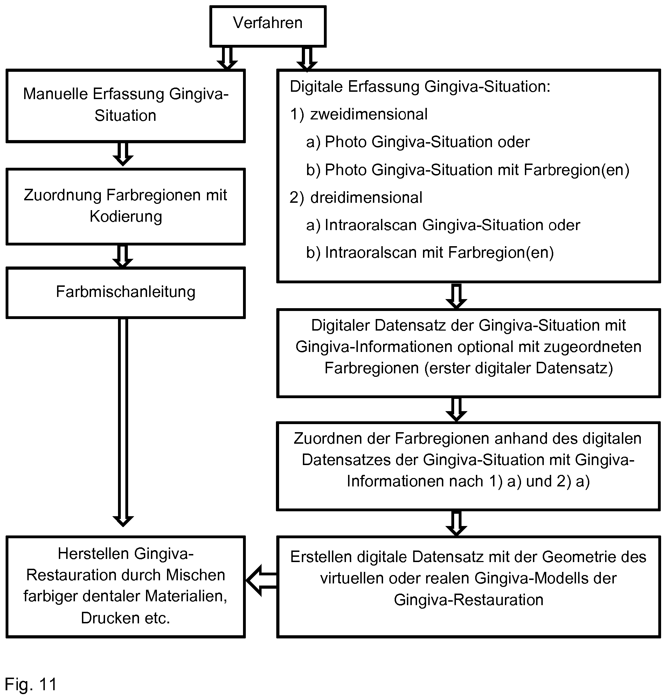

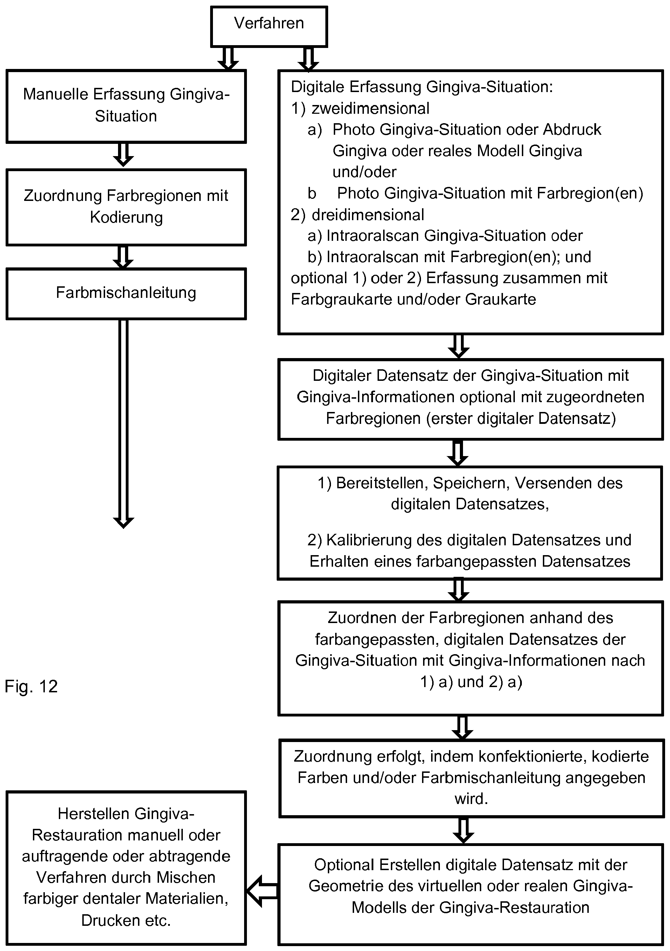

Figur 8 stellt eine mögliche Situation zur Erfassung der Rottöne und deren Farbverläufen dar, indem eine Analyse der Gingiva anhand der Indexierungs-vorrichtung erfolgt und einzelne Farbregionen definierten Positionen der Gingiva zugeordnet werden. Die analysierten und zugeordneten Farbregionen können dann in Bezug auf ihre Position auf die beschriftbare Gingiva-Vorlage der Figuren 9 und 10 übertragen werden. Figur 10 zeigt eine Gingiva-Kartierung der Rottöne und Farbverläufe, d.h. eine mit Gingiva-Informationen beschriftete Gingiva-Vorlage, die über das erfindungsgemäße Verfahren ermittelt wurde. Alternativ kann die Analyse gemäß Figur 8 auch mittels digitaler Verfahren, wie Photographieren oder Intraoralscann erfasst werden. Die einfachste Möglichkeit ist die Erfassung der Gingiva-Situation oder der Gingiva-Situation und Indexierungsvorrichtung mit der Photo- oder Filmfunktion eines Smartphones, eines iPods oder eines ähnlichen Gerätes. Die Speicherung und Weitergabe der Daten kann auf bekannte Art und Weise erfolgen, wie beispielsweise ftp, E-Mail, Speichermedium. Die erhaltenden digitalen Daten des ersten digitalen Datensatzes können dann, wie vorstehend erläutert, weiter bearbeitet werden. Figuren 11 und 12 zeigen mögliche Verfahrensabläufe von erfindungsgemäßen Verfahren zur Erfassung der Gingiva-Situation bis zur Herstellung der Gingiva-Restauration. Figur 12 offenbart das erfindungsgemäße Verfahren unter Verwendung einer Farbgraukarte oder einer Graukarte.

### Bezugszeichenliste:

A: Breite Gingiva-Zahnbogen; B: Höhe Gingiva-Zahnbogen, C: Kontur zwischen den Zähnen; Höhe und Position der Papille, D: Maß Sichtbarkeit Zahnstumpf, E: schwarzes Dreieck, F: Morphologie
- 0: Gingiva-Indexierungsvorrichtung
- 1: Element, insbesondere Flächenelement
- 2: Farbregion, insbesondere farbiger Körper
- 3: Kodierung, kodierte Farbregion
- 4: beschriftbare Gingiva-Vorlage
- 4.1: beschriftbare Gingiva-Vorlage des oberen Frontzahnbereiches,
- 4.2: beschriftbare Gingiva-Vorlage des unteren Frontzahnbereiches,
- 4.3: beschriftbare Gingiva-Vorlage des oberen Seitenzahnbereiches
- 4.4: beschriftbare Gingiva-Vorlage des unteren Seitenzahnbereiches
- 5: übertragene Farbregion
- 6: Gingiva-Restauration (Figur 3)
- 7: Gingiva-Situation (Gingiva Defizit; Zahnfleischrückgang, fehlende Zahnfleischpartie) (Figur 4)
- 8: Basis der Indexvorrichtung und/oder Farbgraukarte und/oder Graukarte

## Patentansprüche

1. Verfahren zur Erfassung der Gingiva-Situation (7) und zur Herstellung eines virtuellen oder realen Modells einer Gingiva-Restauration (6), in dem
A) eine Analyse mindestens eines Bereiches der Gingiva-Situation erfolgt in Bezug auf ihre roten Farbtöne, die Position der roten Farbtöne und/oder den Farbverlauf der roten Farbtöne und optional der Geometrie und/oder der Morphologie (F), wobei die Analyse digital erfolgt, und
B) Erhalten von Gingiva-Informationen,
wobei die Analyse A) als digitale Analyse erfolgt, indem
- mindestens ein Bereich der Gingiva-Situation (7) und optional ein Bereich der Gesichtspartie unter Verwendung einer Farbgraukarte und/oder einer Graukarte zur Kalibrierung erfasst wird, wobei der Bereich der Gingiva zusammen mit der Karte zwei- oder dreidimensional digital erfasst wird, wobei digitale Gingiva-Informationen als digitaler Datensatz erhalten werden, oder
- indem eine zwei- oder dreidimensionale Erfassung mindestens eines Bereiches der Gingiva-Situation (7) und optional der Zahnsituation und optional eines Bereiches der Gesichtspartie und mindestens einer Farbregion (2) mit einem roten Farbton einer Indexierungsvorrichtung erfolgt, wobei die Erfassung zusammen mit der Erfassung mindestens eines Teils einer Farbgraukarte und/oder einer Graukarte zur Kalibrierung erfolgt, wobei digitale Gingiva-Informationen als digitaler Datensatz erhalten werden
C) Herstellen eines virtuellen oder realen Modells einer Gingiva-Restauration.

2. Verfahren nach Anspruch 1, wobei
die Gingiva digital analysiert wird unter Anwendung einer Software, indem die Gingiva-Situation (7) und optional die Zahnsituation mit der Software auf Farbtöne
und/oder Farbverläufe untersucht wird, besonders bevorzugt wird die Gingiva analysiert auf (i) Farbverläufe der Papillen, krestal, im Bereich des Sulcus, zusätzlich oder alternativ wird die (ii) Geometrie der Gingiva analysiert, insbesondere in Bezug auf die Position der Papillen, der krestalen Situation, der Zahnbreite, Alveolarkamm (Kieferkamm), und/oder die Zahnsituation, wie die Sichtbarkeit der Zahnhälse und/oder es wird die (iii) Morphologie (F) der Gingiva analysiert, und
- Erhalten von Gingiva-Informationen.

3. Verfahren nach Anspruch 1 oder 2, **dadurch gekennzeichnet, dass** die Analyse (i) digital unter Anwendung einer Software erfolgt, indem
- anhand der Software ein Weißabgleich und/oder ein Abgleich des Farbstiches in Korrelation zur Farbgraukarte oder Graukarte am digitalen Datensatz der digitalen Gingiva-Informationen erfolgt, und ein farbangepasster digitaler Datensatz erhalten wird,
- der farbangepasste digitale Datensatz der Gingiva-Situation (7) und optional der Zahnsituation wird manuell mit einem Cursor oder automatisiert auf Farbtöne und/oder Farbverläufe analysiert, wobei Gingiva-Informationen erhalten werden, und optional
- werden den Gingiva-Informationen und/oder den jeweiligen Bereichen der Gingiva jeweils unabhängig eine oder mehrere definierte konfektionierte, dentale Farben und/oder eine oder mehrere Farbmischanleitungen zugeordnet.

4. Verfahren nach einem der Ansprüche 1 bis 3, wobei die Gingiva-Informationen in Form einer Gingiva-Kartierung erhalten werden.

5. Verfahren nach einem der Ansprüche 1 bis 4, wobei die Gingiva-Informationen, a) digital kodierten Farbregionen zugeordnet werden wobei unabhängig der Kodierung (3) eine Farbmischanleitung zugeordnet ist

6. Verfahren nach einem der Ansprüche 1 bis 5, **dadurch gekennzeichnet, dass** eine virtuelle oder reale Gingiva-Restauration (6) erstellt wird, wobei
(i) in einem Schritt vor der Erstellung des virtuellen oder realen Gingiva-Modells ein erster digitaler Datensatz umfassend digitale Gingiva-Informationen eines ersten virtuellen Modells der Gingiva-Situation (7) mittels Intraoralscan zusammen mit der Erfassung einer Farbgraukarte oder einer Graukarte zur Kalibrierung und optional mindestens eine Farbregion (2) mit einem roten Farbton einer Indexierungsvorrichtung erstellt wird, alternativ wird ein erster digitaler Datensatz über einen 3D-Scan eines Silikonabdrucks erstellt oder über einen 3D-Scan eines realen Modells und jeweils mit Gingiva-Informationen ergänzt, und optional
(i.1) Durchführen eines Weißabgleichs und/oder eines Abgleichs des Farbstichs und Erhalten eines farbangepassten digitalen Datensatzes, und optional
(i.2) Analyse des Datensatzes auf Farbtöne und/oder Farbverläufe und Erhalten von Gingiva-Informationen, und optional
(i.3) Zuordnen der Gingiva-Informationen zu vorkonfektionierten, dentalen Farben und/oder einer Farbmischanleitung, und
(ii) der digitale Datensatz mit der Geometrie des virtuellen oder realen Gingiva-Modells der Gingiva-Restauration (6) mit Gingiva-Informationen erstellt wird, und (iii) Herstellen einer Gingiva-Restauration (6).

## Claims

1. Method for detecting the gingival situation (7) and for producing a virtual or real model of a gingival restauration (6), in which
A) an analysis of at least one region of the gingival situation is performed with respect to its shades of red, the position of the shades of red and/or the colour gradient of the shades of red and, optionally, the geometry and/or morphology (F), the analysis being performed digitally, and
B) obtaining gingival information,
analysis A) being performed as a digital analysis, by
- at least one region of the gingival situation (7) and, optionally, one region of the face being detected using a colour grey chart and/or a grey chart for calibration, the region of the gingiva being detected digitally in a two- or three-dimensional manner in conjunction with the chart, digital gingival information being obtained as a digital data set, or
- by a two- or three-dimensional detection of at least one region of the gingival situation (7) and, optionally, of the dental situation and, optionally, of a region of the face and at least one colour region (2) having a shade of red of an indexing device being performed, the detection being performed in conjunction with the detection of at least one part of a colour grey chart and/or grey chart for calibration, digital gingival information being obtained as a digital data set
C) producing a virtual or real model of a gingival restauration.

2. Method according to claim 1, wherein
the gingiva is analysed digitally using a software, by the gingival situation (7) and, optionally, the dental situation being examined with the software for colour shades and/or colour gradients, particularly preferably the gingiva being analysed for (i) colour gradients of the papillae, crestal, in the region of the sulcus, additionally or alternatively the (ii) geometry of the gingiva being analysed, in particular with respect to the position of the papillae, the crestal situation, the tooth width, the alveolar ridge (jaw crest), and/or the dental situation, such as the visibility of the dental necks and/or the
(iii) morphology (F) of the gingiva being analysed, and
- obtaining gingival information.

3. Method according to claim 1 or 2, **characterised in that** analysis (i) is performed digitally using a software, by
- a white balance and/or balance of the colour cast in correlation to the colour grey chart or grey chart being performed by means of the software on the digital data set of the digital gingival information, and a colour-adapted digital data set being obtained,
- the colour-adapted digital data set of the gingival situation (7) and, optionally, of the dental situation being analysed manually with a cursor or automatically for colour shades and/or colour gradients, gingival information being obtained, and optionally
- one or more defined assembled, dental colours and/or one or more colour mixing instructions, each independently, being assigned to the gingival information and/or the respective regions of the gingiva.

4. Method according to any one of the claims 1 to 3, wherein the gingival information is obtained in the form of a gingival mapping.

5. Method according to any one of the claims 1 to 4, wherein the gingival information a) is assigned to digitally coded colour regions, a colour mixing instruction being assigned regardless of the coding (3).

6. Method according to any one of the claims 1 to 5, **characterised in that** a virtual or real gingival restoration (6) is prepared,
(i) a first digital data set comprising digital gingival information of a first virtual model of the gingival situation (7) being prepared by means of intraoral scanning in conjunction with the detection of a colour grey chart and/or grey chart for calibration and, optionally, at least one colour region (2) having a red shade of an indexing device in a step prior to preparation of the virtual or real gingival model, alternatively a first digital data set being prepared via a 3D scan of a silicone impression or via a 3D scan of a real model and being supplemented with gingival information respectively, and optionally
(i.1) performing a white balance and/or a balance of the colour cast and obtaining a colour-adapted digital data set, and optionally
(i.2) analysis of the data set for colour shades and/or colour gradients, and obtaining gingival information, and optionally
(i.3) assigning the gingival information to pre-assembled dental colours and/or a colour mixing instruction, and
(ii) the digital data set with the geometry of the virtual or real gingival model of the gingival restoration (6) being prepared with gingival information, and
(iii) producing a gingival restoration (6).

## Revendications

1. Procédé pour détecter la situation gingivale (7) et pour produire un modèle virtuel ou réel d'une restauration gingivale (6), dans lequel
A) une analyse d'au moins une région de la situation gingivale est effectuée par rapport à leurs nuances de rouge, la position des nuances de rouge et/ou le dégradé de couleurs des nuances de rouge et, facultativement la géométrie et/ou morphologie (F), l'analyse étant effectuée numériquement, et
B) obtenir des informations gingivales,
l'analyse A) étant effectuée en tant qu'une analyse numérique, par
- au moins une région de la situation gingivale (7) et, facultativement, une région de la face étant détectée utilisant une charte de gris de couleur et/ou une charte de gris pour la calibration, la région de la gencive étant détectée numériquement de manière deux- ou tridimensionnelle conjointement à la charte, des information gingivales étant obtenues en tant qu'un ensemble de données numérique, ou
- par la détection deux- ou tridimensionnelle d'au moins une région de la situation gingivale (7) et, facultativement, de la situation dentaire et, facultativement, de la région de la face et au moins une région de couleurs (2) ayant une nuance de rouge d'un dispositif d'indexage étant effectuée, la détection étant effectuée conjointement à la détection d'au moins une partie d'une charte de gris de couleur et/ou charte de gris pour la calibration, des informations gingivales numériques étant obtenues en tant qu'un ensemble de données numérique,
C) produire un modèle virtuel ou réel d'une restauration gingivale.

2. Procédé selon la revendication 1, selon lequel
la gencive est analysée numériquement utilisant un logiciel par la situation gingivale (7) et, facultativement, la situation dentaire étant examinée avec le logiciel pour des nuances de couleurs et/ou des dégradés de couleurs, de préférence particulier la gencive étant analysée pour (i) des dégradés de couleurs des papilles, crestale, dans la région du sulcus, additionnellement ou alternativement la (ii) géométrie de la gencive étant analysée, en particulier par rapport à la position des papilles, la situation crestale, la largeur de dents, la crête alvéolaire (la crête de mâchoire), et/ou la situation dentaire, telle que la visibilité des collets de dents et/ou la
(ii) morphologie (F) de la gencive étant analysée, et
- obtenir des informations gingivales.

3. Procédé selon la revendication 1 ou 2, **caractérisé en ce que** l'analyse (i) est effectuée numériquement utilisant un logiciel, par
- une balance de blancs et/ou une balance de la teinte de couleurs en corrélation à la charte de gris de couleur ou charte de gris étant effectuée par moyen du logiciel à l'ensemble de données numérique des informations gingivales numériques, et un ensemble de données numérique à couleurs adaptées étant obtenu,
- l'ensemble de données numérique à couleurs adaptées de la situation gingivale (7) et, facultativement, de la situation dentaire étant analysé manuellement avec un curseur ou automatiquement pour les nuances de couleurs et/ou dégradés de couleurs, des informations gingivales étant obtenues, et facultativement
- une ou plusieurs couleurs dentaires confectionnées définies et/ou un ou plusieurs instructions de mélange à couleurs, chaque indépendamment, étant assignés aux informations gingivales et/ou les régions respectives de la gencive.

4. Procédé selon l'une des revendications 1 à 3, selon lequel les informations gingivales sont obtenues sous la forme d'une cartographie gingivale.

5. Procédé selon l'une des revendications 1 à 4, selon lequel les informations gingivales a) sont assignées à des régions de couleurs encodées numériquement, un instruction de mélange à couleurs étant assigné quel que soit le codage (3).

6. Procédé selon l'une des revendications 1 à 5, **caractérisé en ce qu'**une restauration gingivale (6) virtuelle ou réelle est préparée,
(i) un premier ensemble de données numérique comprenant des informations gingivales d'un premier modèle virtuel de la situation gingivale (7) étant préparé par moyen d'un balayage en bouche conjointement à la détection de la charte de gris de couleur et/ou charte de gris pour la calibration et, facultativement, au moins une région de couleurs (2) ayant un nuance de rouge d'un dispositif d'indexage dans une étape avant la préparation du modèle gingival virtuel ou réel, alternativement un premier ensemble de données étant préparé par un balayage 3D d'une empreinte en silicone ou par un balayage 3D d'un modèle réel et étant supplémenté avec des informations gingivales respectivement, et facultativement
(i.1) effectuer une balance de blancs et/ou une balance de la teinte de couleurs et obtenir un ensemble de données numérique à couleurs adaptées, et facultativement
(i.2) l'analyse de l'ensemble de données pour des nuances de couleurs et/ou des dégradées de couleurs et obtenir des informations gingivales, et facultativement
(i.3) assigner les informations gingivales à des couleurs dentaires preconfectionnées et/ou un instruction de mélange à couleurs, et
(ii) l'ensemble de données numériques avec la géométrie du modèle gingival virtuel ou réel de la restauration gingivale (6) étant préparé avec des informations gingivales, et
(iii) produire une restauration gingivale (6).
